# EUROPEAN PATENT APPLICATION

(11) **EP 4 763 086 A1**
(43) Date of publication of application: **24.06.2026**
(21) Application number: 24847700.2
(22) Date of filing: 13.05.2024
(51) Int. Cl.: A61B 5/293, A61N 1/36

(54) **INTERVENTIONAL DEVICE FOR TUBULAR BODY**

(30) Priority: 01.08.2023 CN 202310962250
(71) Applicant: Shanghai Stairmed Technology Co., Ltd., Shanghai 200131 (CN)
(72) Inventor: YANG, Zhigang, Shanghai 200032 (CN); ZHANG, Shiqiang, Shanghai 200131 (CN)
(74) Representative: Cooley (UK) LLP
(86) International application number: PCT/CN2024/092788
(87) International publication number: WO 2025/025735

(57) **Abstract**

An interventional device for a tubular body includes: a stent switchable between a first state and a second state, where a diameter of the stent in the second state is greater than that in the first state; at least one puncture portion arranged on the stent, where the puncture portion is configured to puncture a wall of the tubular body when the stent is in the second state; and at least one electrode arranged on the at least one puncture portion, where the electrode is configured to extend out of the tubular body from an area of the wall punctured by the puncture portion. By means of the solution of the present disclosure, the effective implantation of the electrode can be achieved on the basis of the interventional device, and the implanted electrode can be in direct contact with a biological tissue target area outside of the tubular body, thereby achieving direct and unobstructed signal interaction and greatly improving the effects of collecting signals and applying stimulation by means of the electrode.

## Description

The present application claims priority to Chinese Patent Application No. 202310962250.2, filed with the China National Intellectual Property Administration on August 01, 2023 and entitled "INTERVENTIONAL DEVICE FOR TUBULAR BODY", which is incorporated herein by reference in its entirety.

### Technical Field

The present invention relates to the field of medical device technologies, and specifically to an interventional device for a tubular body.

### Background

In the field of medical devices, electrodes for sensing electroencephalogram (EEG) signals (such as rigid electrodes and flexible electrodes) are widely studied and applied. Electrodes may be placed close to brain tissue (e.g., on the skull, on the dura mater, or under the dura mater) or implanted into the brain tissue.

The current common approach for implanting an electrode in the brain is first opening the scalp and skull to expose the brain tissue, and then place the electrode on or implant it into the brain tissue. Such an invasive implantation method requires opening the skull to expose brain tissue, which may lead to various adverse reactions and may even affect the health and life safety of the organism.

Another way to sense brain signals or stimulate brain tissue is vascular intervention, which delivers a sensing and/or stimulating device (e.g., an electrode) to a target location via the blood vessel, a natural pathway in the human body. While this enables non-invasive signal collection from brain tissue, EEG signals collected by the implanted electrode are poor in quality and limited in quantity, thereby affecting a signal collection effect of the implanted electrode.

### Summary of the Invention

The technical problem solved by the present invention is to provide an improved interventional device that can achieve effective signal sensing or electrical stimulation of a lesion area or target area with minimal incision in tubular biological tissue, and can achieve good electrode implantation effect.

To solve the above technical problem, embodiments of the present invention provide an interventional device for a tubular body, including: a stent switchable between a first state and a second state, where a diameter of the stent in the second state is greater than that in the first state; at least one puncture portion arranged on the stent, where the puncture portion is configured to puncture a wall of the tubular body when the stent is in the second state; and at least one electrode arranged on the at least one puncture portion, where the electrode is configured to extend out of the tubular body from an area of the wall punctured by the puncture portion.

Optionally, the stent is mesh-shaped, and the at least one puncture portion is located at an intersection of the mesh or on an edge connecting adjacent intersections; and/or, the stent is corrugated, and the at least one puncture portion is located at a different location on the stent.

Optionally, the puncture portion is switchable between a non-erect state and an erect state, where the puncture portion in the erect state extends substantially in a radial direction of the stent.

Optionally, the puncture portion is elastically deformable between the non-erect state and the erect state; and/or, the puncture portion is made of nickel-titanium.

Optionally, a length of the puncture portion in the erect state in an extension direction is greater than a thickness of the wall; and/or, there are a plurality of puncture portions, and a length of at least one puncture portion in the erect state in the extension direction is different from a length of another puncture portion in the erect state in the extension direction.

Optionally, the puncture portion is conical, or at least an end portion of the puncture portion away from the stent has a needle-tip structure.

Optionally, the puncture portion and the stent are integrally formed, or the puncture portion is connected to the stent via a medium, the medium being selected from at least one of an adhesive and a solder.

Optionally, the at least one puncture portion and the at least one electrode are in one-to-one correspondence, or a single puncture portion corresponds to a plurality of electrodes.

Optionally, the electrode includes: a first electrode site, at least arranged at a tip of the puncture portion.

Optionally, the first electrode site is formed by a metal layer covering an outer surface of the puncture portion.

Optionally, a portion of the outer surface of the metal layer is coated with an insulating layer.

Optionally, the interventional device further includes an electrode lead, where the electrode lead is electrically connected to the first electrode site.

Optionally, the electrode lead and the first electrode site are electrically connected by a conductive glue.

Optionally, the electrode includes an electrode wire, the electrode wire including at least one second electrode site, and the puncture portion is adapted to carry at least a portion of the electrode wire to extend out of the tubular body from the area on the wall punctured by the puncture portion.

Optionally, the electrode wire is provided with a hole, where the hole is fitted onto the tip of the puncture portion.

Optionally, the outer surface of the puncture portion is covered with an insulating layer and/or a biodegradable layer.

Optionally, after at least a portion of the electrode wire extends out of the tubular body from the area on the wall punctured by the puncture portion, the stent can return to the first state and be withdrawn from the tubular body.

Optionally, electrode wire holes of a plurality of electrode wires are fitted onto the tip of a same puncture portion.

Optionally, the puncture portion has a cavity, where the cavity is configured to accommodate the electrode wire, the tip of the puncture portion is provided with an opening in communication with the cavity, and the electrode wire can extend out of the puncture portion through the opening.

Optionally, a through hole in communication with the cavity is provided at a connection between the puncture portion and the stent to allow the electrode wire to pass through.

Optionally, the interventional device further includes an electrode wire guiding member accommodated within the cavity, where the electrode wire guiding member is configured to guide the electrode wire to extend out of the puncture portion from the opening.

Optionally, the electrode wire extends along an outer side and/or inner side of the stent to the puncture portion.

Optionally, the interventional device further includes a fixing layer, where a portion of the electrode wire extending along the stent is sandwiched between the fixing layer and the stent.

Optionally, the interventional device further includes an occlusion portion, where the occlusion portion surrounds at least the puncture portion, and is arranged on an outer surface and/or inner surface of the stent, and the occlusion portion is configured to occlude a gap between the wall and the puncture portion.

Optionally, the occlusion portion includes a coating layer.

Optionally, during the first state of the stent, at least the tip of the puncture portion is farther away from the stent than the coating layer.

Optionally, the interventional device further includes a delivery sheath detachably fitted onto the stent in the first state, where the delivery sheath is configured to hold the stent in the first state.

Optionally, an end portion of the delivery sheath near the stent is tearable in a radial direction to be unfolded into a two-dimensional plane.

Optionally, the interventional device further includes a balloon, where the stent is fitted onto the balloon, and the balloon is configured to enable the stent to switch from the first state to the second state.

Optionally, the tubular body includes a blood vessel.

Optionally, a material of the stent is selected from any one or a combination of the following: nickel, titanium, nickel-titanium alloy, cobalt, cobalt alloy, chromium, cobalt-chromium alloy, magnesium, magnesium alloy, zinc alloy, and stainless steel.

Optionally, a material of the puncture portion is selected from any one or a combination of the following: nickel, titanium, nickel-titanium alloy, cobalt, cobalt alloy, chromium, cobalt-chromium alloy, magnesium, magnesium alloy, zinc alloy, and stainless steel.

Optionally, a material of the second electrode site is selected from any one or a combination of the following: gold, platinum, iridium, tungsten, magnesium, molybdenum, platinum-iridium alloy, titanium alloy, graphite, carbon nanotubes, poly(3,4-ethylenedioxythiophene) (PEDOT) and its derivative polymers, poly(p-styrene sulfonic acid), polypyrrole, and iridium oxide.

Optionally, a material of the coating is selected from any one or a combination of the following: cellulose, chitin, hyaluronic acid, collagen, gelatin, sodium alginate, polyurethane (PU), poly tetra fluoroethylene (PTFE), expanded PTFE (E PTFE), polylactic acid (PAL), poly(1 lactic acid) (PLLA), poly(D lactic acid) (PDLLA), polyglycolic acid (PGA), polycaprolactone (PCL), polyamide (PA), polyethylene terephthalate (PET), polyether block polyamide (PEBAX), high-density polyethylene (HDPE), thermoplastic polyurethane elastomer (TPU), polyimide (PI), polydimethylsiloxane (PDMS), poly(para-xylylene) (Parylene), epoxy resin, polyamide-imide (PAI), polylactic acid (PHA), poly(lactic-co-glycolic acid) (PHAH), poly(chloro-p-xylylene), SU8, silica gel, and silicone rubber.

Optionally, a material of the metal layer is selected from any one or a combination of the following: gold, platinum, iridium, tungsten, magnesium, molybdenum, platinum-iridium alloy, and titanium alloy.

Optionally, a material of the insulating layer is selected from any one or a combination of the following: polyimide (PI), polydimethylsiloxane (PDMS), poly(para-xylylene) (parylene), polyethylene terephthalate (PET), epoxy resin, polyamide-imide (PAI), polylactic acid (PHA), poly(lactic-co-glycolic acid) (PHAH), poly(chloro-p-xylylene), SU8, silica gel, and silicone rubber.

Optionally, a material of the biodegradable layer is selected from any one or a combination of the following: polylactic acid (PLA), poly(lactic-co-glycolic acid) (PLGA), and magnesium alloy.

Compared with the prior art, the technical solution of the embodiments of the present invention has the following beneficial effects:
The embodiments of the present invention provide an interventional device for a tubular body, including: a stent switchable between a first state and a second state, where a diameter of the stent in the second state is greater than that in the first state; at least one puncture portion arranged on the stent, where the puncture portion is configured to puncture a wall of the tubular body when the stent is in the second state; and at least one electrode arranged on the at least one puncture portion, where the electrode is configured to extend out of the tubular body from an area of the wall punctured by the puncture portion.

Compared to an existing method where an electrode implanted using an interventional device is attached to an inner wall of a tubular body and interact with a target area through the wall of the tubular body, severely affecting effects of collecting signals and applying stimulation by means of the electrode, this implementation solution enables effective implantation of the electrode on the basis of the interventional device for a tubular body, and the implanted electrode can be in direct contact with a biological tissue target area outside the tubular body, thereby achieving direct and unobstructed signal interaction and greatly improving the effects of collecting signals and applying stimulation by means of the electrode. Specifically, at least one puncture portion is added to the stent. As the stent expands within the tubular body, the puncture portion punctures the wall of the tubular body to form a channel through which the electrode can extend. Furthermore, the electrode, along with the stent, is introduced into the organism through the tubular body and is implanted into the target area near the tubular body via the channel created by the puncture portion. Once the electrode is delivered out of the tubular body through the channel, there is no longer any obstruction from the wall of the tubular body between the electrode and the target area, and therefore, the electrode can directly collect electrical signals from the target area or directly apply stimulation signals to the target area, thereby greatly improving the implantation effect of the electrode.

Furthermore, in a specific application scenario of a brain-computer interface, a neural interface can be established between brain tissue and the interventional device described in this implementation solution to realize the interface between the brain and the machine. Electroencephalogram (EEG) signals are collected or specific electrical signals are provided to stimulate brain regions by an electrode placed or carried on the puncture portion.

Further, the electrode includes: a first electrode site, at least arranged at a tip of the puncture portion. Therefore, by placing the electrode site at the tip of the puncture portion, the electrode site at the tip is simultaneously implanted into the target area while the puncture portion punctures the wall of the tubular body. Furthermore, the first electrode site is formed by a metal layer covering the outer surface of the puncture portion, so as to help the surface of the puncture portion achieve both piercing and implantation functions at the same time. As the stent expands, the electrode implantation action can be completed in one step.

Further, the electrode includes an electrode wire, the electrode wire including at least one second electrode site, and the puncture portion is adapted to carry at least a portion of the electrode wire to extend out of the tubular body from the area on the wall punctured by the puncture portion. Therefore, by carrying the electrode wire through the puncture portion, the electrode wire can pass through the wall of the tubular body to reach outside the tubular body, thereby achieving direct contact with the target area. Furthermore, electrode wires prepared using any existing processes can be carried by the puncture portion to complete the implantation, which helps improve the compatibility of the interventional device described in this implementation solution and enhance its adaptability to various types of electrode wires.

### Brief Description of the Drawings

FIG. 1 is a schematic view of an interventional device for a tubular body according to a first embodiment of the present invention;
FIG. 2 is a schematic view of a stent in FIG. 1 in a first state;
FIG. 3 is a schematic view of the stent in FIG. 1 in a second state;
FIG. 4 is a schematic view of a variation of the stent in FIG. 1;
FIG. 5 is a schematic cross-sectional view of a puncture portion according to a second embodiment of the present invention;
FIG. 6 is a schematic cross-sectional view of a puncture portion according to a third embodiment of the present invention;
FIG. 7 is a partial enlarged view of an electrode wire in FIG. 6;
FIG. 8 is a schematic cross-sectional view of a puncture portion according to a fourth embodiment of the present invention;
FIG. 9 is a schematic cross-sectional view of a variation of the puncture portion shown in FIG. 8;
FIG. 10 is a schematic cross-sectional view of a puncture portion according to a fifth embodiment of the present invention;
FIG. 11 is a schematic view of a delivery sheath according to a sixth embodiment of the present invention;
FIG. 12 is a partial enlarged view of a region A in FIG. 11; and
FIG. 13 is a cross-sectional view of FIG. 1 in a B-B direction.

### Detailed Description

As mentioned in the background, existing electrode implantation using vascular intervention methods can only collect electroencephalogram (EEG) signals of poor quality and in very limited quantity.

The latest research result is a Stentrode (stent electrode recording array) electrode, whose main working principle is that: a disc-shaped electrode is placed on an intracranial stent, the intracranial stent expands in the blood vessel, and the disc-shaped electrode is attached to the inner wall of the blood vessel. An electroencephalogram (EEG) signal sensed by the electrode is a mixture of signals from numerous neurons in the brain tissue around the blood vessel.

These electrodes, implanted via vascular intervention, can only collect brain signals through the blood vessel wall. The electrode pads located inside blood vessels are blocked by the blood vessel walls and cerebrospinal fluid from the neurons in the brain tissue near the blood vessels. As a result, the electrode pads do not directly perform signal collection or stimulation on the neurons, and the EEG signals that the electrode pads can collect are of poor quality and in very limited quantities. The EEG signals collected by the electrode pads are mixed signals from numerous neurons in the brain tissue around the blood vessel. These mixed signals from numerous cannot characterize signal characteristics of specific neurons, nor can they clearly characterize functional features of brain regions. Furthermore, distinguishing these mixed signals from neurons is extremely difficult. At the same time, after analysis and decoding, very little information derived from these EEG signals can be used for amyotrophic lateral sclerosis (ALS), and such signals can only be applied to a small number of behavioral controls, such as moving and confirming a mouse cursor in conjunction with an eye-tracking device.

To solve the above technical problem, embodiments of the present invention provide an interventional device for a tubular body, including: a stent switchable between a first state and a second state, where a diameter of the stent in the second state is greater than that in the first state; at least one puncture portion arranged on the stent, where the puncture portion is configured to puncture a wall of the tubular body when the stent is in the second state; and at least one electrode arranged on the at least one puncture portion, where the electrode is configured to extend out of the tubular body from an area of the wall punctured by the puncture portion.

By using this implementation solution, the effective implantation of the electrode can be achieved on the basis of the interventional device, and the implanted electrode can be in direct contact with a biological tissue target area outside the tubular body, thereby achieving direct and unobstructed signal interaction and greatly improving the effects of collecting signals and applying stimulation by means of the electrode. Specifically, at least one puncture portion is added to the stent. As the stent expands within the tubular body, the puncture portion punctures the wall of the tubular body to form a channel through which the electrode can extend. Furthermore, the electrode, along with the stent, is introduced into the organism through the tubular body and is implanted into the target area near the tubular body via the channel created by the puncture portion. Once the electrode extends out of the tubular body through the channel, there is no longer any obstruction from the wall of the tubular body between the electrode and the target area, and therefore, the electrode can directly collect electrical signals from the target area or directly apply stimulation signals to the target area, thereby greatly improving the implantation effect of the electrode.

To make the above objectives, features, and beneficial effects of the present invention more apparent and comprehensible, specific embodiments of the present invention are described in detail below with reference to the accompanying drawings. The same number is used to label the same part in each figure. The embodiments are merely illustrative, and of course, structures shown in different embodiments can be partially replaced or combined. In the modified examples, descriptions of matters identical to those in the first embodiment are omitted, and only the differences are explained. In particular, the same effects produced by the same structure will not be mentioned one by one in each embodiment.

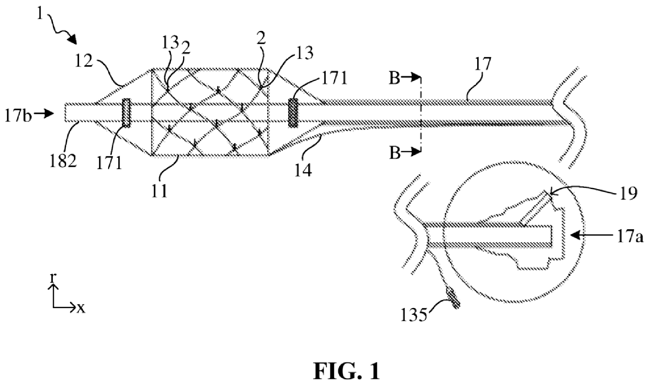

FIG. 1 is a schematic view of an interventional device 1 for a tubular body (briefly referred to as an interventional device 1 in the following) according to a first embodiment of the present invention.

The interventional device 1 may include a device configured to sense and/or stimulate tissue. For example, the interventional device 1 may integrate the device configured to sense and/or stimulate tissue. In other words, the interventional device 1 described in this implementation solution can itself be regarded as a device configured to sense and/or stimulate tissue. For another example, the interventional device 1 can carry the device configured to sense and/or stimulate tissue. After the device configured to sense and/or stimulate tissue, such as an electrode, is implanted into a target area via a tubular body through an interventional procedure, the interventional device 1 may be withdrawn from the tubular body, at which point the electrode is left in the target area.

The tissue may be, for example, cells (e.g., neurons) of an organism (e.g., the brain).

The device configured to sense and/or stimulate tissue may be, for example, an electrode. In some embodiments, the electrode may include a flexible electrode. The flexibility of the flexible electrode may be specifically reflected in the fact that the electrode has a flexible structure that can be bent/folded as needed. The definition of the flexible electrode can refer to the general definition applicable to this field.

The tubular body may be, for example, a blood vessel, trachea, esophagus, renal tubule, laryngeal tube, catheter, and the like.

Based on this implementation solution, the cells of the organism to which the interventional device 1 is intervened interact with the outside world through the electrode to achieve electrical signal interaction. The interventional device 1 can implant electrodes into the target area via a tubular body through an interventional procedure, so that the electrodes can sense and/or stimulate the tissue in the target area. The target area is located outside the tubular body. The tissue surrounding the tubular body into which the interventional device 1 can be intervened can be designated as the target area.

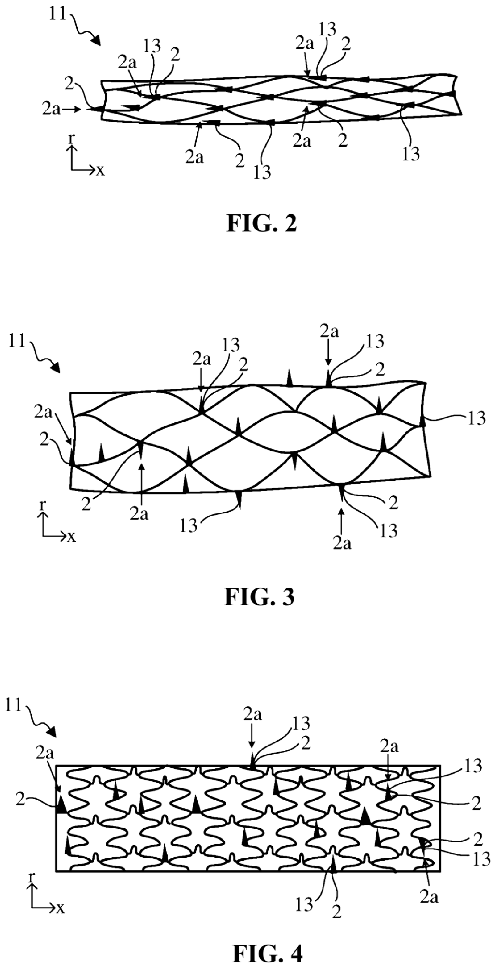

Specifically, referring to FIG. 1 to FIG. 3, the interventional device 1 for a tubular body described in this embodiment may include a stent 11 switchable between a first state (as shown in FIG. 2) and a second state (as shown in FIG. 1 and FIG. 3). A diameter of the stent 11 in the second state is greater than the diameter in the first state.

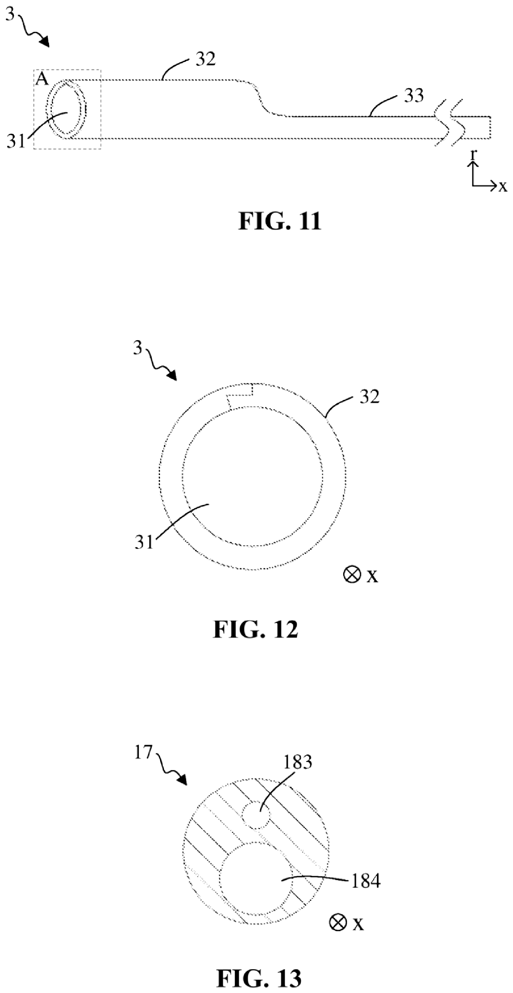

The stent 11 can also be referred to as a support frame. For example, the stent 11 may have at least a contracted state (e.g., the first state shown in FIG. 2), a released state, and an expanded state (e.g., the second state shown in FIG. 1 and FIG. 3). In some embodiments, the stent 11 can be restricted to the contracted state by an external force, and enter the released state as the external force is removed, and further enter the expanded state under its own deformation and/or the action of another external force. The external force may come from, for example, a delivery sheath 3 fitted onto the stent 11 (as shown in FIG. 11), and the other external force may come from, for example, a balloon 12 (refer to the balloon stent in the prior art). In one specific embodiment, the stent 11 may be of an overall ring structure, such as a ring-shaped corrugated structure.

For ease of description, a radial direction of the stent 11 is denoted as an r direction, and an axial direction of the stent 11 is denoted as an x direction. The stent 11 has an inner surface 11a and an outer surface 11b that are opposite to each other in the radial direction. An inner side corresponding to the inner surface 11a is a surface facing the balloon 12, and an outer side corresponding to the outer surface 11b is a surface facing a wall of the tubular body.

Furthermore, continuing to refer to FIG. 1 to FIG. 3, the interventional device 1 may further include at least one puncture portion 2 arranged on the stent 11, the puncture portion 2 being configured to puncture the wall of the tubular body when the stent 11 is in the second state.

For example, the puncture portion 2 may be fixed to the outer surface 11b of the stent 11 and extend substantially outward in the radial direction (e.g., the r direction) at least when the stent 11 is in the second state. Furthermore, one end of the puncture portion 2 far away from the stent 11 is a tip 2a to ensure reliable puncture of the wall of the tubular body.

For another example, a plurality of puncture portions 2 may be distributed at different locations on the stent 11, which helps increase contact points with the wall of the tubular body.

Furthermore, the interventional device 1 may also include at least one electrode 13 arranged at at least one puncture portion 2, the electrode 13 being configured to extend out of the tubular body from the area punctured by the puncture portion 2 on the wall. The electrode 13 is a part for establishing a neural interface on the corresponding puncture portion 2.

Furthermore, the electrode 13, extending out of the tubular body from the area punctured by the puncture portion 2, reaches the target area (e.g., brain tissue) near the periphery of the tubular body, thereby establishing a direct connection with, for example, brain neurons.

Furthermore, each electrode 13 has a corresponding electrode lead 14, and FIG. 1 exemplarily shows one of the electrode leads 14. In some embodiments, each electrode lead 14 corresponds to at least one electrode 13. For example, the electrode leads 14 and the electrodes 13 are in one-to-one correspondence.

Furthermore, the interventional device 1 may also include a balloon 12, the stent 11 is fitted onto the balloon 12, and the balloon 12 is configured to enable the stent 11 to switch from the first state to the second state. For example, the balloon 12 and the stent 11 fitted thereon can be collectively referred to as a balloon stent, and the balloon stent has at least a contracted state, a released state, and an expanded state. As an implant, the balloon stent can move in an inner cavity of the tubular body to a target location.

In a typical application scenario, a balloon stent in the contracted state is delivered to the target location through an interventional procedure. Then, the balloon stent is released and gas or liquid is injected into the balloon 12 to open the stent 11, thereby causing the stent 11 to enter the expanded state, as shown in FIG. 1. Under the action of the expansion force, the puncture portion 2 on the stent 11 can puncture the wall of the tubular body, and the tip 2a of the puncture portion 2 can extend out of the tubular body, thereby implanting the electrode 13 into the target area near the periphery of the tubular body.

In some embodiments, in the axial direction (e.g., the x direction), the stent 11 has a tubular shape that matches the shape of the blood vessel. The stent 11 has a self-supporting performance due to material properties of the metal it is made of, and is retractable in both its axial direction (e.g., the x direction) and radial direction (e.g., the r direction).

In some embodiments, the stent 11 may be made of a highly elastic alloy such as nickel-titanium.

In some embodiments, the stent 11 may be manufactured by cutting, carving, metal weaving, and the like.

In some embodiments, the stent 11 is in the released state after being released from the delivery sheath 3 that wraps it, and in the expanded state after being subsequently deployed by the inflated balloon 12. In the released state and the expanded state, the morphology of the stent 11 is mesh-like (as shown in FIG. 1 and FIG. 3) or corrugated (as shown in FIG. 4). When viewed in the radial direction thereof, the entire stent 11 presents a porous structure (as shown in FIG. 1 and FIG. 3) or a striped structure (as shown in FIG. 4).

Taking the tubular body being a blood vessel and the target area being brain tissue as an example, the stent 11 is first delivered to the target location by the delivery sheath 3 in the delivery process, and then the stent 11 is released and exposed in the blood vessel by withdrawing the delivery sheath 3. At this point, the stent 11 is in the released state and has a certain degree of self-expansion property.

Then, by inflating the balloon 12 inside the stent 11 with gas or liquid, the stent 11 is further expanded and deployed. The stent 11 is expanded and opened under the dual action of the self-expansion and the inflation of the balloon 12, so that the puncture portion 2 on the stent 11 punctures the blood vessel and comes into contact with the brain tissue outside the blood vessel, thereby allowing the electrode 13 located at the puncture portion 2 to come into contact with the brain tissue outside the blood vessel, and further enabling the sensing of brain signals or the application of electrical stimulation. For example, the electrode 13 can be arranged at the tip 2a of puncture portion 2. For another example, the tip 2a of the puncture portion 2 may carry the electrode 13.

Therefore, the puncture portion 2 is provided on the stent 11, and the puncture portion 2 can puncture the tubular body (e.g., the blood vessel). The electrode 13 is also provided on the puncture portion 2. After the puncture portion 2 punctures the tubular body, the electrode 13 can be in contact with the target area (e.g., the brain tissue) outside the tubular body, thereby enabling the direct collection of electrical signals (e.g., EEG signals) from the target area or the transmission of stimulation signals to the target area.

In some embodiments, the number of puncture portions 2 provided on the stent 11 may be any number, for example, 1, 2, 4, 6, 8, 16, 20, 24, 36, 40, 60, 64, or the like.

In one specific implementation, referring to FIG. 1 to FIG. 3, the stent 11 may be in the form of a mesh. Furthermore, at least one puncture portion 2 may be arranged at a fulcrum of the stent 11 or at another location that can provide a support force. The fulcrum may be, for example, an intersection point of the support frame.

For example, at least one puncture portion 2 may be distributed at an intersection of the mesh or on an edge connecting adjacent intersections.

In some embodiments, a spacing between adjacent intersections of the mesh-shaped stent 11 in the first state may be smaller than a spacing between adjacent intersections of the mesh-shaped stent 11 in the second state.

In one variation, referring to FIG. 4, the stent 11 may be corrugated. Furthermore, at least one puncture portion 2 may be distributed at different locations on the stent 11. For example, locations of crests, troughs, and locations between the crests and troughs of the corrugations can all be configured to arrange the puncture portions 2. The electrode leads 14 connecting the electrodes 13 are not shown in FIG. 2 to FIG. 4.

In one specific implementation, continuing to refer to FIG. 1 to FIG. 3, the puncture portion 2 is switchable between a non-erect state (as shown in FIG. 2) and an erect state (as shown in FIG. 3 and FIG. 1), with the puncture portion 2 in the erect state extending substantially in the radial direction (e.g., the r direction) of the stent 11.

Specifically, referring to FIG. 2, the non-erect state can be, for example, a lying posture, where an included angle between an extension direction of the puncture portion 2 (e.g., a direction from one end connected to the stent 11 to the tip 2a) and the axial direction of the stent 11 is essentially zero. Specifically, the included angle may refer to an included angle between the extension direction of the puncture portion 2 and a supporting surface that provides support for the puncture portion 2. It should be noted that in practical applications, a specific value of the included angle in the non-erect state can be reasonably determined within a range of [0, 90) degrees according to the specific shape of the tubular body (e.g., the degree of curvature, the diameter, and the like) to facilitate the implantation of the interventional device 1 and avoid the tip 2a of the puncture portion 2 from scratching the tubular body.

Furthermore, the erect state can include an approximately erect state. As the stent 11 enters the released state, the puncture portion 2 changes from the non-erect state as shown in FIG. 2 to the erect state or approximately erect state, thereby maximizing the support force of the stent 11 on it during the expansion process of the stent 11. This facilitates the puncture portion 2 to puncture the wall of the tubular body under the support of the expansion force of the stent 11. It should be noted that in practical applications, the specific value of the included angle in the erect state can be reasonably determined within the range of [0, 90) degrees according to the specific shape of the tubular body (e.g., the degree of curvature, the diameter, and the like) to ensure that the tip 2a of the puncture portion 2 in the erect state can smoothly puncture the wall of the tubular body along with the expansion of the stent 11.

In some embodiments, the puncture portion 2 may be made of a metal with good elasticity, such as nickel-titanium or its alloy, so as to provide elasticity to the puncture portion 2. This enables the puncture portion 2 to have a certain degree of flexibility and allows it to self-restore to the erect state. In other words, the puncture portion 2 can elastically deform between the non-erect state and the erect state.

Specifically, when the delivery sheath 3 (that is, a cannula of the entire interventional device 1) wrapping the stent 11 is withdrawn, the elasticity of the puncture portion 2 allows it to return to the erect state or approximately erect state. At this point, a central axis of the puncture portion 2 has a corresponding angle (e.g., preferably 90 degrees) with its supporting surface on the stent 11, so that the puncture portion 2 can obtain a support force based on the supporting surface on the stent 11 to carry out the subsequent tubular puncture operation.

In some embodiments, provided that the puncture portion 2 in the erect state is sufficient to puncture the wall of the tubular body, the switching of the puncture portion 2 between the non-erect state and the erect state may not be limited to elastic deformation. For example, the puncture portion 2 may extend and retract in the radial direction (e.g., in the r direction) to switch between the non-erect state and the erect state.

In a typical application scenario, during the delivery of the interventional device 1 into a tubular biological tissue, such as a blood vessel, the stent 11, together with the puncture portion 2, is retracted into the delivery sheath 3. Upon reaching the target location, the delivery sheath 3 is withdrawn, the stent 11 is released, and the puncture portion 2 is also released. At this point, the puncture portion 2 can automatically reopen to an erect state.

In one specific implementation, a length of the puncture portion 2 in the extension direction, which is in the erect state, is greater than a thickness of the wall of the tubular body. This ensures that the puncture portion 2 in the erect state can puncture the wall, so that the electrode 13 can extend to the outside of the tubular body. Furthermore, by rationally designing the length of the puncture portion 2, after puncturing the blood vessel, the puncture portion 2 has a certain margin to intervene in brain tissue, thereby ensuring that the electrode 13 can effectively establish a direct electrical connection with the brain nerves.

In some embodiments, the length of the puncture portion 2 in the extension direction can be determined based on the thickness of the wall of the tubular body to be implanted by the interventional device 1.

In one specific implementation, there are a plurality of puncture portions, and the length of at least one puncture portion 2 in the erect state in the extension direction may be different from the length of another puncture portion 2 in the erect state in the extension direction.

For example, the lengths of the puncture portions 2 in the erect state provided in different areas on the outer periphery of the stent 11 may be different.

For another example, the puncture portions 2 of different lengths may be alternately and evenly distributed on the outer surface 11b of the stent 11.

Therefore, it can be compatible with tubular bodies with different wall thicknesses, allowing the interventional device 1 to be adapted to different types of tubular bodies.

In some embodiments, for a plurality of puncture portions 2 that have different lengths in the erect state, the lengths of the puncture portions 2 in the non-erect state may be the same in the extension direction. This could be beneficial for facilitating the delivery of the interventional device 1 within the tubular body.

In some embodiments, by rationally arranging the distribution of a plurality of puncture portions 2 on the stent 11, damages caused by collisions between adjacent puncture portions 2 when the stent 11 is in the first state can be avoided.

Furthermore, when the stent 11 is in a first location, adjacent puncture portions 2 can be stacked one on top of the other.

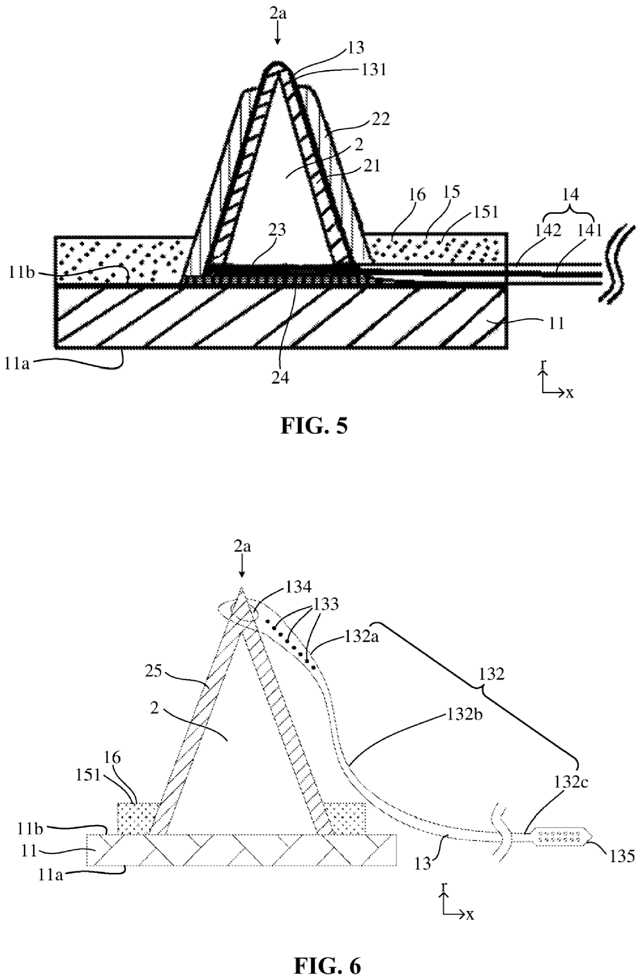

In one specific implementation, the puncture portion 2 may be cone-shaped, as shown in FIG. 5 to FIG. 9. For example, it can have a narrow, long, conical structure. The tip of the cone is suitable for forming the tip 2a.

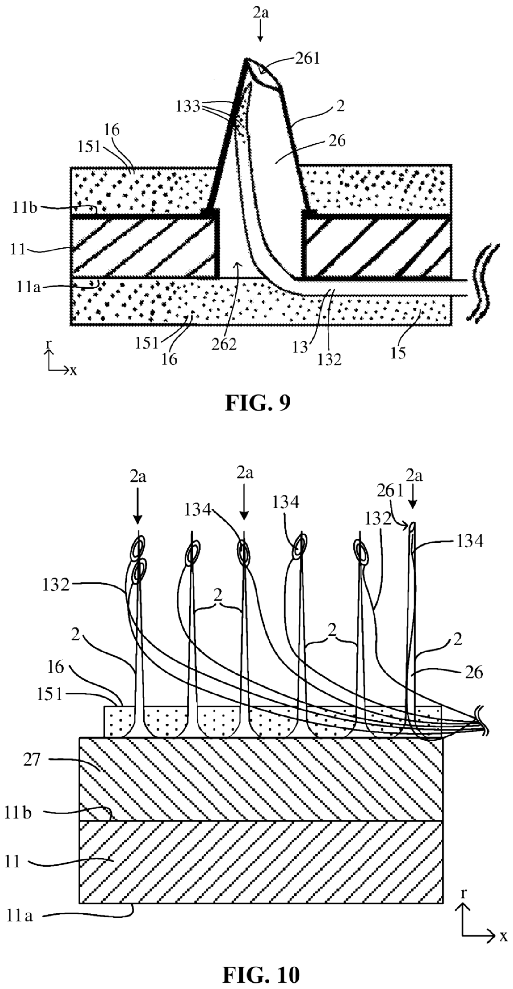

In one variation, at least the end portion of the puncture portion 2 away from the stent 11 is a needle-tip structure, as shown in FIG. 10, to form the tip 2a sufficient to puncture the wall of the tubular body.

In some embodiments, the puncture portion 2 may be manufactured by a processing technique such as etching.

In one specific implementation, the puncture portion 2 can be fixed to the stent 11 by means of, for example, bonding, laser welding, or the like. Specifically, the puncture portion 2 can be connected to the stent 11 via a medium, and the medium may be at least one selected from an adhesive and a solder (also known as a welding agent). The adhesive may be, for example, a glue, such as a medical glue. The welding agent may be, for example, a laser welding agent.

In one variation, the puncture portion 2 may be integrally formed with the stent 11. This helps improve the overall structural strength of the stent 11 and the puncture portion 2 arranged on the stent 11. Furthermore, the stent 11 can better provide a support force for the puncture portion 2 to ensure that the puncture portion 2 can quickly and reliably puncture the wall of the tubular body.

In one specific implementation, a single puncture portion 2 may correspond to at least one electrode 13.

For example, at least one puncture portion 2 may be in one-to-one correspondence with at least one electrode 13. That is, each puncture portion 2 is provided with or carries one electrode 13.

For example, a single puncture portion 2 may correspond to a plurality of electrodes 13. That is, one puncture portion 2 may carry or be equipped with a plurality of electrodes 13 at the same time, which is beneficial to increase the number of electrodes 13 that can establish a direct connection with the target area after the puncture portion 2 punctures the wall of the tubular body.

In some embodiments, a single puncture portion 2 may be provided with a plurality of tips 2a.

For example, referring to FIG. 10, a single puncture portion 2 may include a plurality of needle-tip structures, where each needle-tip structure can carry one or a plurality of electrodes 13 (e.g., electrode wires 132). The plurality of needle-tip structures may be arranged in an array on a base 27, and the base 27 may be fixed to the outer surface 11b of the stent 11. The needle-tip structure may be hollow and in communication with an internal hollow region at the tip 2a. The tip 2a is designed with a bevel to ensure that it can puncture the wall of the tubular body. Alternatively, the needle-tip structure may be solid, with the tip 2a fitted in a hole 134 of the electrode wire 132, so as to carry the electrode 13 on the needle-tip structure.

For example, a single puncture portion 2 may include a plurality of cone-shaped structures, with one of the cone-shaped structures as a main body, forming a tree-like branching structure. Each cone-shaped structure may carry or be provided with one electrode 13.

In one specific embodiment, referring to FIG. 5, the electrode 13 may include a first electrode site 131, which is at least arranged at the tip 2a of the puncture portion 2. In this implementation solution, different functional structures are distinguished by different types of fill in each exemplary cross-sectional view.

Specifically, the tip 2a may be, for example, the end portion of the puncture portion 2 in the erect state that is away from the stent 11.

Furthermore, each first electrode site 131 may be adapted to form an electrode 13, and one first electrode site 131 is provided at each tip 2a of each puncture portion 2. In other words, the first electrode site 131 and the tip 2a are in one-to-one correspondence.

In an application where the target area around the tubular body is brain tissue, the first electrode site 131 may be configured to sense brain signals or apply electrical stimulation to the brain tissue.

Therefore, by placing the electrode site at the tip 2a of the puncture portion 2, the electrode site at the tip 2a is simultaneously implanted into the target area while the puncture portion 2 punctures the wall of the tubular body.

In some embodiments, the tip 2a itself may form the first electrode site 131. For example, the puncture portion 2 is made, entirely or at least at the tip 2a, of a conductive material to form the first electrode site 131.

In some embodiments, the first electrode site 131 may be formed by a metal layer 21 covering the outer surface of the puncture portion 2.

Specifically, the upper surface of the puncture portion 2 (including the tip 2a) may have a metal coating (e.g., the metal layer 21). The additionally coated metal layer 21 helps improve the conductivity of the surface of the puncture portion 2.

In some embodiments, the material of the metal layer may be, for example, any one or a combination of gold, platinum, iridium, tungsten, magnesium, molybdenum, platinum-iridium alloy, and titanium alloy.

Therefore, the surface layer of the puncture portion 2 may simultaneously achieve the functions of puncture and implantation and site signal collection. With the deployment of the stent 11, the implantation of the electrode 13 may be completed in one step.

Furthermore, the material selection for the puncture portion 2 itself is more flexible and is not limited to conductive materials. For example, the material of the puncture portion 2 may be selected from the perspective such as elasticity and hardness in the erect state. This helps reduce costs and allows for obtaining a harder puncture portion 2 to ensure puncture reliability. Furthermore, nickel-titanium may be used to make the puncture portion 2 to balance elasticity and conductivity. Furthermore, the metal layer 21 made of tungsten, gold, or another material may cover the outer surface of the puncture portion 2 to further enhance the conductivity.

In one specific implementation, continuing to refer to FIG. 5, a portion of the outer surface of the metal layer 21 may be coated with an insulating layer 22.

Specifically, the outer surface of the metal layer 21, except for the tip 2a, may be coated with the insulating layer 22.

Furthermore, the insulating layer 22 can protect the metal layer 21 from scratches and can help ensure the insulation between the area of the puncture portion 2 other than the electrode site 131 and the surrounding environment and other puncture portions 2.

In some embodiments, a material of the insulating layer 22 may be selected from any one or a combination of the following: polyimide (PI), polydimethylsiloxane (PDMS), poly(para-xylylene) (parylene), polyethylene terephthalate (PET), epoxy resin, polyamide-imide (PAI), polylactic acid (PHA), poly(lactic-co-glycolic acid) (PHAH), poly(chloro-p-xylylene), SU8, silica gel, and silicone rubber.

In some embodiments, the size of the area of the metal layer 21 exposed outside the insulating layer 22 is adapted to ensure that, after the tip 2a punctures the wall of the tubular body, the exposed area can be reliably electrically connected to the cells at the target location.

In some embodiments, while the puncture portion 2 is in the non-erect state, the metal layer 21 and the insulating layer 22 attached to the surface of the puncture portion 2 may also be in a deformed state.

In one specific implementation, continuing to refer to FIG. 5, the electrode lead 14 may be electrically connected to the first electrode site 131.

Specifically, the electrode leads 14 may be in one-to-one correspondence with the first electrode sites 131, thereby transmitting signals between the corresponding first electrode sites 131 and the outside world (e.g., computing devices) without interference.

For example, the puncture portion 2 is cone-shaped, one puncture portion 2 corresponds to one cone, and one first electrode site 131 is provided at the tip of each cone. Correspondingly, each cone is connected to an independent electrode lead 14.

Furthermore, the electrode lead 14 may include a wire core 141 and a wire sheath 142 covering the wire core 141. The wire core 141 may serve as an electrical connection, and the conductor sheath 142 may serve as insulation, isolation and protection.

In one specific implementation, referring to FIG. 5, the electrode lead 14 and the first electrode site 131 may be electrically connected by a conductive glue.

Specifically, the conductive glue may be applied between the puncture portion 2 and the stent 11.

For example, the puncture portion 2 is coated at least at the bottom with a conductive adhesive (e.g., the conductive glue) to form a conductive adhesive layer 23 between the bottom surface and the stent 11. The conductive adhesive layer 23 is electrically connected not only to the metal layer 21, but also to the electrode lead 14.

Furthermore, the puncture portion 2 may be connected to the stent 11 by the conductive adhesive and a fixing adhesive. For example, a fixing adhesive layer 24, the conductive adhesive layer 23, and the puncture portion 2 are sequentially stacked on the stent 11 from the inside to the outside in the radial direction (e.g., the r direction). In other words, the conductive adhesive is placed between the puncture portion 2 and the fixing adhesive.

During preparation, the fixing adhesive may be first applied to the stent 11 to form the fixing adhesive layer 24, then the conductive adhesive may be applied to the fixing adhesive layer 24 to form the conductive adhesive layer 23, and finally the puncture portion 2 is fixed on the conductive adhesive layer 23 and allows the conductive adhesive to be connected to the electrode lead 14. In this way, the conductive adhesive can not only adhere the puncture portion 2 to the stent 11 together with the fixation adhesive, but also further transmit the EEG signal collected by the first electrode site 131 on the puncture portion 2 to the electrode lead 14 through the conductive adhesive layer 23, or transmit the stimulation signal transmitted from the electrode lead 14 to the first electrode site 131 on the tip 2a to provide electrical stimulation to the brain tissue.

In some embodiments, the electrode lead 14 may be connected to a location at the bottom or side of the puncture portion 2. For example, it may be connected to the conductive glue at the bottom or side of the puncture portion 2.

In some embodiments, a projected area of the fixed adhesive layer 24 on the stent 11 may be greater than a projected area of the conductive adhesive layer 23 on the stent 11. Furthermore, the insulating layer 22 may cover a portion of the fixing adhesive layer 24 that is not covered by the puncture portion 2 (including the metal layer 21 covering the puncture portion 2), the conductive adhesive layer 23, and the electrode lead 14.

In one variation, the conductive glue may be applied around the connection between the puncture portion 2 and the fixing adhesive layer 24 (i.e., the side of the puncture portion 2), and the end portion of the electrode lead 14 may be adhered to the area coated with the conductive glue to achieve electrical connection.

Specifically, the fixing adhesive layer 24 and the puncture portion 2 are sequentially stacked on the stent 11 from the inside to the outside in the radial direction (e.g., the r direction). Furthermore, the conductive adhesive is applied around a connection gap between the fixing adhesive layer 24 and the puncture portion 2 (the outer surface of which is coated with the metal layer 21) to electrically connect the metal layer 21 and the electrode lead 14.

Furthermore, the insulating layer 22 may cover the outer surface of the metal layer 21, excluding the tip 2a, and the outer surface of the conductive adhesive.

In one specific embodiment, referring to FIG. 6 to 10, the electrode 13 may include an electrode wire 132, and the electrode wire 132 may include at least one second electrode site 133. Cells in the target area achieve signal interaction with the outside world by the second electrode site 133 through the electrode wire (e.g., wiring and a back-end portion 135 within the electrode wire). In some embodiments, the back-end portion 135 may be, for example, a signal processing device.

Specifically, the electrode wire 132 may be a flexible structure that can be bent/folded as needed. Furthermore, the electrode wire 132 may be, for example, an ultra-flexible electrode wire with a micrometer-scale size.

Furthermore, at least one second electrode site 133 may be provided at an end portion of the electrode wire 132, where each second electrode site 133 and the back-end portion 135 are electrically connected through the wiring within the electrode wire.

Furthermore, the puncture portion 2 may be adapted to carry at least a portion of the electrode wire 132 to extend out of the tubular body from the area on the wall of the tubular body punctured by the puncture portion 2.

Therefore, by carrying the electrode wire 132 through the puncture portion 2, the electrode wire 132 can pass through the wall of the tubular body to reach outside the tubular body, thereby achieving direct contact with the target area. Furthermore, electrode wires 132 prepared using any existing processes can be carried by the puncture portion 2 to complete the implantation, which helps improve the compatibility of the interventional device 1 described in this implementation solution and enhance its adaptability to various types of electrode wires 132.

In some embodiments, at least one second electrode site 133 may be arranged at intervals in the extension direction of the electrode wire 132. Each of the second electrode sites 133 may independently establish a direct electrical connection with cells in the target area.

In some embodiments, the electrode wire 132 may be fabricated using the microelectromechanical processing (MEMS) technology. Specifically, the electrode wire 132 may include the second electrode site 133 and a lead structure (e.g., wiring inside the electrode wire).

The second electrode site 133 is an exposed metal site, also known as an electrode contact.

The lead structure is adapted to form the electrode lead 14 for transmitting EEG signals sensed or collected by the second electrode site 133 to a back-end device (e.g., the back-end portion 135), or for transmitting stimulation signals transmitted from the back-end device to brain tissue cells.

Furthermore, the second electrode site 133 is typically made of a low-resistivity metal such as gold or platinum.

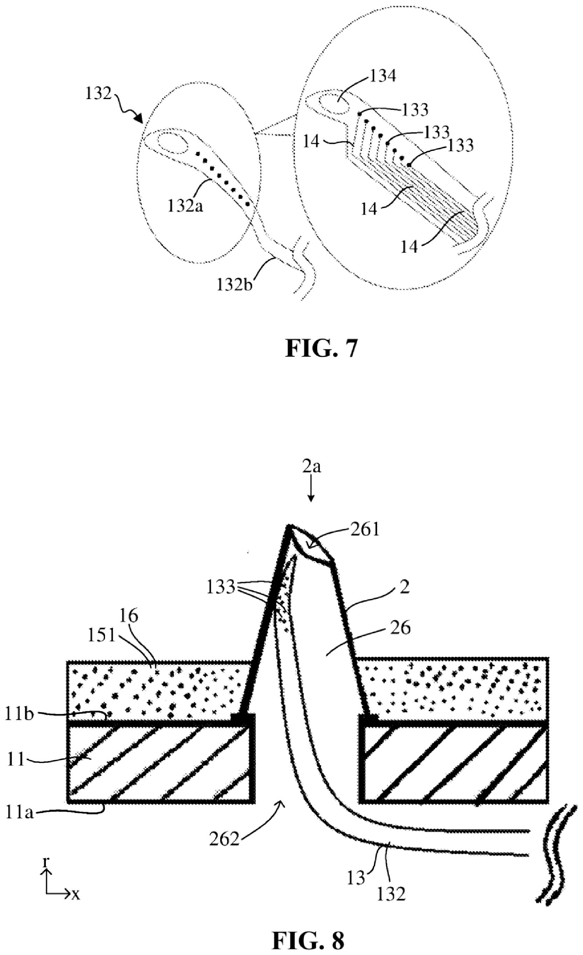

For example, referring to FIG. 7, the second electrode site 133 is in one-to-one correspondence with the electrode lead 14. The electrode lead 14 is configured to transmit the EEG signals sensed or collected by the corresponding second electrode site 133 or to transmit the stimulation signals transmitted from the back-end device to neurons in the brain tissue. The electrode leads 14 are separated by an insulating material.

Furthermore, referring to FIG. 6 and FIG. 7, in the extension direction, the electrode wire 132 may include a site segment 132a at a front end, a lead segment 132b in the middle, and a back-end segment 132c at the back end. The site segment 132a is at least partially implanted into the target area (e.g., the brain tissue) outside the tubular body to sense EEG signals or to deliver stimulation signals. The lead segment 132b is at least partially attached to the stent 11 (e.g., the coating layer 151 covering the stent 11) to fix the electrode wire 132, which helps stabilize the signal transmission. The back-end segment 132c may be located outside the organism.

In some embodiments, a main difference between the puncture portion 2 in the embodiment shown in FIG. 6 to FIG. 10 and the puncture portion 2 in the embodiment shown in FIG. 5 is that the puncture portion 2 in the embodiment shown in FIG. 6 to FIG. 10 may not play (or not primarily play) a conductive role, and the electrical connection with the biological tissue in the target area is achieved by the electrode wire 132 carried outside the tubular body by the tip 2a of the puncture portion 2.

In one specific implementation, referring to FIG. 6, FIG. 7, and FIG. 10, the electrode wire 132 may be carried outside the tubular body in the form of being fitted onto the puncture portion 2.

Specifically, the puncture portion 2 may be fixedly connected to the stent 11. For example, the fixed connection may be achieved by adhesive bonding or welding, and the welding may be, for example, laser welding.

Furthermore, the electrode wire 132 may be provided with the hole 134 (also referred to as a wire hole).

For example, the hole 134 may be located at the very front end of the electrode wire 132. That is, compared to at least one second electrode site 133, the hole 134 may be closer to the end portion of the electrode wire 132.

For another example, the hole 134 may be located in the middle of the section where at least one second electrode site 133 of the electrode wire 132 is located.

Furthermore, the hole 134 may be fitted onto the tip 2a of the puncture portion 2.

For example, the tip 2a of the cone-shaped puncture portion 2 passes through the hole 134, thereby carrying the electrode wire 132 into the target area as the tip 2a punctures the wall of the tubular body and enters the target area (e.g., the brain tissue) outside the tubular body.

Furthermore, after the electrode wire 132 is implanted into the target area such as the brain tissue, the second electrode site 133 on the electrode wire 132 can come into contact with the neurons in the brain tissue, thereby being capable of directly obtaining brain signals from the neurons and directly transmitting stimulation signals to the neurons. In this way, EEG signals from specific brain regions can be directly sensed or collected. The EEG signals are of better quality and have fewer mixed signals, which facilitates subsequent signal processing and analysis, and even allows control of an external device based on the EEG signals.

In some embodiments, the electrode wire 132 may also be connected to the tip 2a by means of adhesion so that it can be implanted into the target area as the tip 2a is inserted into the target area.

Therefore, by adopting this implementation solution, after the puncture portion 2 on the stent 11 carries the electrode wire 132 to be implanted into the target area, the second electrode site 133 on the electrode wire 132 can collect electrical signals from the target area or provide electrical stimulation to the target area.

Furthermore, after implementation of the electrode wire 132 is completed, the stent 11 may remain on the inner wall of the tubular body and maintain the implantation state of the puncture portion 2.

Alternatively, the stent 11 along with the puncture portion 2 may be withdrawn from the tubular body, leaving only the electrode wire 132 in the target area. For example, after at least a portion of the electrode wire 132 extends out of the tubular body from the area on the wall of the tubular body punctured by the puncture portion 2, the stent 11 may return to the first state and be withdrawn from the tubular body.

In some embodiments, the outer surface of the puncture portion 2 may be covered with a coating 25. The coating 25 may form a protective layer on the surface of the puncture portion 2, and may further reduce the potential damage of the metal tip 2a to the hole 134 when the hole 134 is fitted onto the tip 2a.

In some embodiments, the coating 25 may be, for example, the insulating layer 22. A difference from the puncture portion 2 described in the embodiment shown in FIG. 5 above is that, in this example, the insulating layer 22 can completely cover the outer surface of the puncture portion 2, that is, the tip 2a of the puncture portion 2 is also covered by the insulating layer 22.

In some embodiments, the coating 25 may be, for example, a biodegradable layer. Specifically, the biodegradable layer may be composed of a biodegradable material, and the biodegradable material may include a polymeric material such as polylactic acid (PLA) and poly(lactic-co-glycolic acid) (PLGA), as well as a biodegradable metallic material such as magnesium alloy. Furthermore, after the electrode wire 132 is implanted into the target area, the biodegradable layer may be further degraded, thereby reducing the risk of damage to the target area and facilitating the separation of the puncture portion 2 and the electrode wire 132.

In some embodiments, the coating 25 may be, for example, the insulating layer 22 and the biodegradable layer. For example, the insulating layer 22 may cover the outer surface of the puncture portion 2, and the outer surface of the insulating layer 22 may be further coated with the biodegradable layer.

In some embodiments, each puncture portion 2 may be provided with one or a plurality of electrode wires 132. For example, holes 134 of the plurality of electrode wires 132 may be fitted onto the tip 2a of a same puncture portion 2.

In one specific implementation, referring to FIG. 8 and FIG. 9, the electrode wire 132 may be carried outside the tubular body in the form of being accommodated in the puncture portion 2. In other words, the difference from the embodiments shown in FIG. 6 and FIG. 10 above is that, in this embodiment, the electrode wire 132 of the interventional device 1 does not contact with the wall of the tubular body during the puncture portion 2 puncturing the wall of the tubular body, but extends out of the tubular body through the interior of the stent 11 and the interior of the puncture portion 2.

Specifically, the puncture portion 2 may have a cavity 26, and the cavity 26 is configured to accommodate the electrode wire 132.

Furthermore, the tip 2a of the puncture portion 2 has an opening 261 in communication with the cavity 26, and the electrode wire 132 can extend out of the puncture portion 2 from the opening 261. Thus, as the tip 2a punctures the wall of the tubular body and enters the target area, the electrode wire 132 can be implanted into the target area outside the tubular body along with the tip 2a.

In some embodiments, one or a plurality of electrode wires 132 may be accommodated within the cavity 26 of a single puncture portion 2.

In some embodiments, a cross section of the opening 261 at the tip 2a may be a slope, that is, the cross section may not be perpendicular to the radial direction (e.g., the r direction) of the stent 11. Thus, the tip 2a with the opening 261 is shaped like a needle tip. While puncturing the wall of the tubular body, the electrode wire 132 accommodated in the cavity 26 is transported from the opening 261 to the tip 2a in a manner similar to needle injection, so as to achieve electrical connection with the cells in the target area outside the tubular body.

In some embodiments, a bottom end of the puncture portion 2 may be fixed to the stent 11 by adhesive bonding or welding.

In one specific implementation, a through hole 262 in communication with the cavity 26 may be provided at the connection between the puncture portion 2 and the stent 11, for the electrode wire 132 to pass through.

Specifically, the connection may be located, for example, at the bottom of the puncture portion 2. Correspondingly, the through hole 262 extends in the radial direction (e.g., in the r direction) to communicate the cavity 26 and the interior of the stent 11, and the electrode wire 132 extends along an inner side of the stent 11 to the puncture portion 2. Specifically, the inner side of the stent 11 may refer to a side facing the balloon 12.

For example, the through hole 262 may be formed by opening a hole directly below the cavity 26 in the radial direction (e.g., the r direction). The through hole 262 is in communication with the cavity 26, so that the electrode wire 132 can extend from the stent 11 into the cavity 26 of the puncture portion 2 through the through hole 262. In other words, the electrode wire 132, such as the lead section 132b, can extend along the inner surface 11a of the stent 11.

In one variation, the connection may be located, for example, on a side surface of the puncture portion 2. For example, an opening may be provided on the side surface of the puncture portion 2 near the stent 11 to allow the electrode wire 132 to pass through. Correspondingly, the through hole 262 extends in the axial direction (e.g., in the x direction) of the stent 11 to communicate the cavity 26 with the outer surface 11b of the stent 11, and the electrode wire 132 extends along an outer side of the stent 11 to the puncture portion 2. Specifically, the outer side of the stent 11 may refer to a side facing the wall of the tubular body.

In some embodiments, the electrode wire 132 may be movable relative to the stent 11.

Furthermore, when at least the stent 11 is in the first state, the front ends of the electrode wire 132 (e.g., at least the site segment 132a) can abut against the inner wall of the cavity 26. After the tip 2a of the puncture portion 2 punctures the tubular body and enters the target area, the electrode wire 132 can extend from the cavity 26 through the opening 261 and be implanted into the target area.

In one specific implementation, the interventional device 1 may further include an electrode wire guiding member (not shown) accommodated in the cavity 26, and the electrode wire guiding member guides the electrode wire 132 to extend out of the puncture portion 2 through the opening 261.

In one specific implementation, continuing to refer to FIG. 5 and FIG. 9, the interventional device 1 may further include a fixing layer 15, with a portion of the electrode lead 14 or electrode wire 132 extending along the stent 11 sandwiched between the fixing layer 15 and the stent 11.

Specifically, the fixing layer 15 may be, for example, the coating layer 151. The electrode lead 14 or electrode wire 132 may extend between the stent 11 and the coating layer 151.

Furthermore, the coating layer 151 may be attached to (e.g., in close contact with) the outer surface 11b and/or the inner surface 11a of the stent 11.

Before use (e.g., when the stent 11 is in the first state), the tip 2a of the puncture portion 2 passes through the coating layer 151 in advance. In other words, during the first state of the stent 11, at least the tip 2a of the puncture portion 2 is farther away from the stent 11 than the coating layer 151.

In a typical application scenario, when the stent 11 is in the delivery sheath 3, it is in an unreleased state (i.e., the contracted state), and at this point, the puncture portion 2 on the stent 11 is also in an unreleased state. At this stage, the puncture portion 2 has already penetrated the coating layer 151.

In the unreleased state, the puncture portion 2 may lie on the surface of the coating layer 151. In the subsequent released state, the puncture portion 2 changes from the lying state (e.g., the non-erect state) to the erect state so as to obtain the support force of puncturing the tubular body from the surface of the stent 11.

In some embodiments, the front end of the electrode wire 132 may have a certain amount of margin that is not sandwiched between the fixing layer 15 and the stent 11, so as to ensure that after the tip 2a punctures the wall of the tubular body, the front end of the electrode wire 132 can move further out of the tubular body relative to the tip 2a, so as to ensure successful implantation into the target area and establishment of an electrical connection with the biological tissue in that area.

For example, the portion of the electrode wire 132 accommodated within the cavity 26 may be bent at least once to allow for some margin, and this portion of the electrode wire 132 may extend beyond the tip 2a from the opening 261 after the tip 2a punctures the wall of the tubular body.

In one specific implementation, continuing to refer to FIG. 5 to FIG. 10, the interventional device 1 may further include an occlusion portion 16 arranged at least around the puncture portion 2 on the outer surface 11b and/or inner surface 11a of the stent 11, the occlusion portion 16 being configured to occlude a gap between the wall of the tubular body and the puncture portion 2.

In one specific implementation, the interventional device 1 may further include the occlusion portion 16, where the occlusion portion 16 surrounds at least the puncture portion 2, and is arranged on the outer surface 11b and/or inner surface 11a of the stent 11, and the occlusion portion 16 is configured to occlude the gap between the wall of the tubular body and the puncture portion 2.

Specifically, the occlusion portion 16 may include the coating layer 151, which may be made of a highly biocompatible medical coating material such as PTFE.

Furthermore, the coating layer 151 may be manufactured using various processing methods such as stretching and electrospinning. An upper surface of the coating layer 151 (e.g., a surface facing the wall of the tubular body) may have a high specific surface area (the specific surface area refers to a total area per unit mass of material), thereby enabling it to have high adhesion to biological tissue.

The function of the coating layer may be sealing and stopping bleeding on the wound of the tubular body after the puncture portion 2 punctures the tubular body and implants it into the biological tissue of the target area, while also enabling the stent 11 to quickly achieve endothelialization on the inner wall of the tubular body.

In some embodiments, the coating layer 151 may exist only in the area where each puncture portion 2 is located. For example, the coating layer 151 may surround the area where the bottom end of the puncture portion 2 is located, so as to selectively attach to and cover the tiny wound created by the tip 2a of the puncture portion 2 piercing the wall of the tubular body.

In some embodiments, the coating layer 151 may cover the entire inner surface 11a and/or outer surface 11b of the stent 11.

In some embodiments, the coating layer 151 may be, for example, a single-layer film, as shown in FIG. 5, FIG. 6, FIG. 8, and FIG. 10.

In some embodiments, the coating layer 151 may also be two or more layers of film. For example, a main difference between the embodiment shown in FIG. 9 and the embodiment shown in FIG. 8 is that the interventional device 1 shown in FIG. 9 has a coating layer 151 arranged on both the inner surface 11a and the outer surface 11b of the stent 11.

Specifically, the puncture portion 2 passes through the coating layer 151 located on the outer surface 11b of the stent 11 in advance. The coating layer 151 located on the outer surface 11b of the stent 11 can seal the tiny wound caused when the tip 2a punctures the wall of the tubular body.

Furthermore, the coating layer 151 located on the inner surface 11a of the stent 11 can be configured to close the through hole 262 of the stent 11 on the side of the tubular body to prevent fluid (e.g., blood in the blood vessel) in the tubular body from entering the cavity 26 of the puncture portion 2 through the through hole 262 and then flowing out of the tubular body through the opening 261 of the puncture portion 2, causing internal bleeding.

Furthermore, an electrode unit, which includes the electrode wire 132 and the electrode wire guiding member, may extend between the stent 11 and the coating layer 151 located on the inner surface 11a of the stent 11 in advance, and enter the cavity 26 through the through hole 262. Thus, the electrode unit can be fixed between the stent 11 and the coating layer 151. At this point, the coating layer 151 located on the inner surface 11a of the stent 11 may serve as the aforementioned fixing layer 15.

Furthermore, in the example where the tubular body is a blood vessel, regardless of whether the blood vessel is a vein or an artery, the coating layer 151 located on the inner surface 11a of the stent 11 can provide a good seal and apply a certain pressure to the electrode unit on the blood vessel side, which is beneficial for the fixation of the electrode unit between the stent 11 and the coating layer 151, and also beneficial for the subsequent implantation of the electrode unit.

In some embodiments, the occlusion portion 16 may also be made of another hemostatic material.

In some embodiments, the stent 11 may be located between the coating layer 151 and the electrode wire 132 (or the electrode lead 14) in the radial direction (e.g., the r direction) of the stent 11, as shown in FIG. 8.

In some embodiments, the coating layer 151 may be located between the stent 11 and the electrode wire 132 (or the electrode lead 14) in the radial direction (e.g., the r direction) of the stent 11, as shown in FIG. 6.

In one specific implementation, referring to FIG. 11 and FIG. 12, the interventional device 1 may further include the delivery sheath 3 configured to deliver the balloon stent to the target location of the tubular body. The balloon stent may include the balloon 12 and the stent 11 fitted onto the balloon 12.

Specifically, the delivery sheath 3 is detachably fitted onto the stent 11 in the first state to hold the stent 11 in the first state.

Furthermore, the delivery sheath 3 can protect the balloon stent provided with the puncture portion 2 from damage during delivery. Furthermore, the delivery sheath 3 is able to overcome the resistance within the tubular body to successfully deliver the balloon stent structure to the target location.

Furthermore, the balloon 12 and the stent 11 of the balloon stent are superimposed radially, that is, the two are in a concentric circle structure, with the stent 11 surrounding the balloon 12 in the radial direction (e.g., the r direction). An inner diameter of the delivery sheath 3 may be equal to or greater than an outer diameter of the balloon 12 and the stent 11 that are superimposed radially in the contracted state.

Furthermore, the radially superimposed balloon 12 and stent 11 may be arranged within the delivery sheath 3 in advance. For example, the delivery sheath 3 may be provided with an inner cavity 31 configured to accommodate the balloon stent.

The inner cavity 31 may extend in the axial direction (e.g., the x direction) of the stent 11, and the delivery sheath 3 may have a uniform diameter.

Furthermore, the delivery sheath 3 may also have a sleeve section 32 and a delivery section 33, where a diameter of the sleeve section 32 is greater than a diameter of the delivery section 33. In the x direction, the sleeve section 32 is arranged at the front end to accommodate the main body part of the balloon stent, such as the part where the balloon 12 and stent 11 are located. The delivery section 33 is arranged at the back end to accommodate the back end portion of the balloon stent, such as the portion of a catheter 17.

In some embodiments, the delivery sheath 3 may be, for example, a metal spring tube or a metal braided tube.

In some embodiments, the delivery sheath 3 may have a polymer coating.

In some embodiments, the softness of the delivery sheath 3 gradually decreases from the front end to the back end.

In one specific embodiment, continuing to refer to FIG. 11 and FIG. 12, the end portion (e.g., the front end) of the delivery sheath 3 near the stent 11 may be torn in the radial direction (e.g., in the r direction) to be unfolded into a two-dimensional plane.

Specifically, the delivery sheath 3 has a tubular structure with a tearable and deployable front end, as shown in FIG. 11.

Furthermore, the tubular structure with a tearable and deployable front end can be opened and removed from the front end after the delivery sheath 3 is withdrawn. This avoids damage to the electrode lead 14 and its back-end portion, as well as the back-end portion of the balloon 12.

For example, when the delivery sheath 3 is withdrawn to a catheter back end 17a (as exemplarily shown in the circle in FIG. 1), it may be torn open from the front end and removed directly from the catheter 17, making it more convenient for the user.

In one variation, the delivery sheath 3 may be a tubular structure with a complete front end.

In one specific implementation, continuing to refer to FIG. 1 and FIG. 13, the interventional device 1 may also include a balloon unit, and the balloon unit may include at least a balloon 12, a catheter 17 (also referred to as a balloon catheter), and a guide wire 182.

Specifically, the catheter 17 may have at least one lumen.

For example, referring to FIG. 13, the catheter 17 may include a first lumen 183 and a second lumen 184, which are isolated from each other. Both the first lumen 183 and the second lumen 184 extend in the axial direction (e.g., the x direction) of the catheter 17. The first lumen 183 is in communication with the inner cavity of the balloon 12. The second lumen 184 serves as a channel for the movement of the guide wire 182.

In some embodiments, the balloon 12 may be located at a front end 17b of the catheter 17, and the inner cavity of the balloon 12 may be in communication with the first lumen 183 of the catheter 17. Gas, saline, contrast fluid, and the like may be filled into the balloon 12 through the first lumen 183.

In a typical application scenario, referring to FIG. 1, when the stent 11 is in the contracted state (for example, the first state shown in FIG. 2), the balloon 12 is also in a contracted state and is wrapped by the stent 11. As the balloon 12 and stent 11 move within the delivery sheath 3 in with the guide wire 182, both the balloon 12 and stent 11 are in the contracted state, the stent 11 is attached to the surface of the balloon 12 in a manner of wrapping around the balloon 12, and the two move together within the delivery sheath 3 in fixed relative locations. During this period, the puncture portion 2 passes through the coating layer 151 in advance and is in a non-erect state.

After reaching the target location within the tubular body, the balloon 12 and stent 11 are released. At this point, the balloon 12 is inflated by filling the balloon 12 with gas, saline, contrast fluid, or the like.

When the balloon 12 is in the inflated state, the balloon 12 expands and provides an expansion force to the stent 11 that wraps the balloon 12, thereby causing the stent 11 to enter the expanded state. During this period, the puncture portion 2 deforms to the erect state.

When the stent 11 is in the expanded state (e.g., the second state shown in FIG. 1 and FIG. 3), the stent 11 is closely attached to the inner wall of the blood vessel and supports the tip 2a of the puncture portion 2 to puncture the blood vessel, thereby enabling the tip 2a to be implanted into the brain tissue surrounding the blood vessel.

Furthermore, the back end 17a of the catheter is provided with an opening for the guide wire 182 to be inserted. The opening is in communication with the second lumen 184. The guide wire 182 is inserted into the second lumen 184 through the opening and extends to the front end 17b of the catheter 17. The front end 17b of the catheter 17 is closed. When delivering the balloon stent into a human blood vessel, the guide wire 182 can guide the entire catheter 17.

Furthermore, the balloon 2 may exist within the delivery sheath 3 in a folded structure with two, three, or five folds before being filled. The two, three, or five folds may be similar to folding methods used in folding umbrellas.

Furthermore, the electrode lead 14 may be wired along the inner surface 11a of the stent 11 or along the outer surface 11b of the stent 11 to electrically connect the corresponding electrode 13 to the external device.

In a typical application scenario, given tortuous and flexible natures of intracranial blood vessels, both the delivery sheath 3 and the catheter 17 need to have higher permeability and delivery capability. In interventional treatment, the target location is first determined by angiography, and then the delivery sheath 3 is delivered into the blood vessel from the femoral vein, jugular vein, femoral artery or brachial artery. The catheter 17 is accommodated in the delivery sheath 3. When the delivery sheath 3 reaches the target blood vessel location, the guide wire 182 also guides the catheter 17 along the pathway to the target location.

After both the delivery sheath 3 and the catheter 17 have reached the target location, the delivery sheath 3 is withdrawn to release the balloon 12 and the stent 11 from the delivery sheath 3. At this point, the stent 11 is in the released state, and the puncture portion 2 on the stent 11 is in the erect state.

Then, an inflating seat (e.g., an inflating port 19) of the back end 17a of the catheter is connected to an inflating device (not shown), and an appropriate volume of contrast fluid (or saline) is filled into the cavity of the balloon 12 through the first lumen 183, causing the balloon 12 to expand, thereby further expanding the stent 11 to enter the expanded state.

During the expansion of the stent 11, the expansion force of the stent 11 causes the puncture portion 2 on the stent 11 to puncture through the blood vessel wall and enter the brain tissue surrounding the blood vessel.

After the stent 11 is pressed into the inner wall of the blood vessel, the tip 2a of the puncture portion 2, together with the electrode wire 132 set thereon, is also implanted into the brain tissue surrounding the blood vessel.

Then, the balloon 12 is depressurized, so that the balloon 12 is changed from an inflated state to a negative pressure state, and the catheter 17 is then withdrawn from the body.

In some embodiments, during the implantation of the interventional device 1, a real-time location of the balloon 12 may be displayed by a visualization ring 171 arranged on the catheter 17.

For example, there may be two visualization rings 171, which are respectively located on both sides of the stent 11 in the x direction.

In some embodiments, the diameter of the catheter 17 is about 0.5 mm to 1 mm, and its length in the x direction is about 50 mm to 150 mm.

In some embodiments, the length of the balloon 12 in the x direction is approximately 0.5 mm to 15 mm.

In some embodiments, the diameter of the stent 11 before expansion is approximately 0.5 mm to 1.0 mm, and the diameter after expansion is approximately 1.5 mm to 2.0 mm.

Therefore, by using this implementation solution, the effective implantation of the electrode 13 can be achieved on the basis of the interventional device, and the implanted electrode 13 can be in direct contact with a biological tissue target area outside the tubular body, thereby achieving direct and unobstructed signal interaction and greatly improving the effects of collecting signals and applying stimulation by means of the electrode. Specifically, at least one puncture portion 2 is added to the stent 11. As the stent 11 expands, the puncture portion 2 punctures the wall of the tubular body to form a channel through which the electrode 13 can extend. Furthermore, the electrode 13, along with the stent 11, is introduced into the organism through the tubular body and is implanted into the target area near the tubular body via the channel created by the puncture portion 2. Once the electrode 13 is delivered out of the tubular body through the channel, there is no longer any obstruction from the wall of the tubular body between the electrode and the target area, and therefore, the electrode 13 can directly collect electrical signals from the target area or directly apply stimulation signals to the target area, thereby greatly improving the implantation effect of the electrode 13.

Furthermore, in a specific application scenario of a brain-computer interface, a neural interface can be established between brain tissue and the interventional device 1 described in this implementation solution to realize the interface between the brain and the machine. EEG signals are collected or specific electrical signals are provided to stimulate brain regions by the electrode 13 placed or carried on the puncture portion 2.

Furthermore, the coating layer 151 is added to the stent 11, and the tip 2a of the puncture portion 2 passes through the coating layer 151 in advance. Thus, after the tip 2a punctures the blood vessel, the coating layer 151 can quickly adhere to the resulting wound, thereby preventing the blood vessel from bleeding and helping repair the wound of the blood vessel.

In this implementation solution, the material of the coating layer is selected from any one of the following materials: cellulose, chitin, hyaluronic acid, collagen, gelatin, sodium alginate, polyurethane (PU), poly tetra fluoroethylene (PTFE), expanded PTFE (E PTFE), polylactic acid (PAL), poly(1 lactic acid) (PLLA), poly(D lactic acid) (PDLLA), polyglycolic acid (PGA), polycaprolactone (PCL), polyamide (PA), polyethylene terephthalate (PET), polyether block polyamide (PEBAX), high-density polyethylene (HDPE), thermoplastic polyurethane elastomer (TPU), polyimide (PI), polydimethylsiloxane (PDMS), poly(para-xylylene) (Parylene), epoxy resin, polyamide-imide (PAI), polylactic acid (PHA), poly(lactic-co-glycolic acid) (PHAH), poly(chloro-p-xylylene), SU8, silica gel, and silicone rubber.

Nickel-titanium alloys possess properties such as shape memory effect, temperature control, superelasticity, fatigue resistance, and good biocompatibility, making them suitable as medical materials for clinical applications in fields such as dentistry, gynecology, orthopedics, and cardiovascular.

Medical nickel-titanium alloys are metals primarily configured to manufacture implantable medical devices. The nickel-titanium alloy is a shape memory alloy that can automatically recover to its original state within a specific temperature range after undergoing plastic deformation. The shape memory metal has advantages such as good biocompatibility and fast stress recovery. In the medical field, the shape memory metal suitable for implantation in the human body is mainly nickel-titanium alloy because it has good biocompatibility and its properties can be changed by adjusting its composition. The shape memory metal is particularly well-suited for use in in-body stents, such as cardiac stents, which are often used as a treatment for vascular diseases. The cardiac stents generally use nickel-titanium alloys.

In the implementation solution of the present invention, the material of the stent is selected from any one or a combination of the following: nickel, titanium, nickel-titanium alloy, cobalt, cobalt alloy, chromium, cobalt-chromium alloy, magnesium, magnesium alloy, zinc alloy, and stainless steel.

In the implementation solution of the present invention, the material of the puncture portion is selected from any one or a combination of the following: nickel, titanium, nickel-titaniumalloy, cobalt, cobalt alloy, chromium, cobalt-chromium alloy, magnesium, magnesium alloy, zinc alloy, and stainless steel.

In the implementation solution of the present invention, the material of the electrode site is selected from any one or a combination of the following: gold, platinum, iridium, tungsten, magnesium, molybdenum, platinum-iridium alloy, titanium alloy, graphite, carbon nanotubes, poly(3,4-ethylenedioxythiophene) (PEDOT) and its derivative polymers, poly(p-styrene sulfonic acid), polypyrrole, and iridium oxide.

In the implementation solution of the present invention, the material of the metal layer is selected from any one or a combination of the following: gold, platinum, iridium, tungsten, magnesium, molybdenum, platinum-iridium alloy, and titanium alloy.

In the implementation solution of the present invention, the material of the insulating layer is selected from any one or a combination of the following: polyimide (PI), polydimethylsiloxane (PDMS), poly(para-xylylene) (Parylene), polyethylene terephthalate (PET), epoxy resin, polyamide-imide (PAI), polylactic acid (PHA), poly(lactic-co-glycolic acid) (PHAH), poly(chloro-p-xylylene), SU8, silica gel, and silicone rubber.

In the implementation solution of the present invention, the material of the biodegradable layer is selected from any one or a combination of the following: polylactic acid (PLA), poly(lactic-co-glycolic acid) (PLGA), and magnesium alloy.

Although the present invention is disclosed as above, it is not limited thereto. Any person skilled in the art may make various changes and modifications without departing from the spirit and scope of the present invention. Therefore, the protection scope of the present invention shall be subject to the scope defined by the claims.

## Claims

1. An interventional device for a tubular body, comprising:
a stent switchable between a first state and a second state, wherein a diameter of the stent in the second state is greater than that in the first state;
at least one puncture portion arranged on the stent, wherein the puncture portion is configured to puncture a wall of the tubular body when the stent is in the second state; and
at least one electrode arranged on the at least one puncture portion, wherein the electrode is configured to extend out of the tubular body from an area of the wall punctured by the puncture portion.

2. The interventional device according to claim 1, wherein the stent is mesh-shaped, and the at least one puncture portion is located at an intersection of the mesh or on an edge connecting adjacent intersections; and/or, the stent is corrugated, and the at least one puncture portion is located at a different location on the stent.

3. The interventional device according to claim 1, wherein the puncture portion is switchable between a non-erect state and an erect state, and the puncture portion in the erect state extends substantially in a radial direction of the stent.

4. The interventional device according to claim 3, wherein the puncture portion is elastically deformable between the non-erect state and the erect state; and/or, the puncture portion is made of nickel-titanium.

5. The interventional device according to claim 3, wherein a length of the puncture portion in the erect state in an extension direction is greater than a thickness of the wall; and/or, there are a plurality of puncture portions, and a length of at least one puncture portion in the erect state in the extension direction is different from a length of another puncture portion in the erect state in the extension direction.

6. The interventional device according to claim 1, wherein the puncture portion is conical, or at least an end portion of the puncture portion away from the stent has a needle-tip structure.

7. The interventional device according to claim 1, wherein the puncture portion and the stent are integrally formed, or the puncture portion is connected to the stent via a medium, the medium being selected from at least one of an adhesive and a solder.

8. The interventional device according to claim 1, wherein the at least one puncture portion and the at least one electrode are in one-to-one correspondence, or a single puncture portion corresponds to a plurality of electrodes.

9. The interventional device according to claim 1, wherein the electrode comprises: a first electrode site, at least arranged at a tip of the puncture portion.

10. The interventional device according to claim 9, wherein the first electrode site is formed by a metal layer covering an outer surface of the puncture portion.

11. The interventional device according to claim 10, wherein a portion of the outer surface of the metal layer is coated with an insulating layer.

12. The interventional device according to claim 9, further comprising an electrode lead, wherein the electrode lead is electrically connected to the first electrode site.

13. The interventional device according to claim 12, wherein the electrode lead and the first electrode site are electrically connected by a conductive glue.

14. The interventional device according to claim 1, wherein the electrode comprises an electrode wire, the electrode wire comprising at least one second electrode site, and the puncture portion is adapted to carry at least a portion of the electrode wire to extend out of the tubular body from the area on the wall punctured by the puncture portion.

15. The interventional device according to claim 14, wherein the electrode wire is provided with a hole, and the hole is fitted onto the tip of the puncture portion.

16. The interventional device according to claim 15, wherein the outer surface of the puncture portion is covered with an insulating layer and/or a biodegradable layer.

17. The interventional device according to claim 15, wherein after at least a portion of the electrode wire extends out of the tubular body from the area on the wall punctured by the puncture portion, the stent is capable of returning to the first state and being withdrawn from the tubular body.

18. The interventional device according to claim 15, wherein electrode wire holes of a plurality of electrode wires are fitted onto the tip of a same puncture portion.

19. The interventional device according to claim 14, wherein the puncture portion has a cavity, the cavity is configured to accommodate the electrode wire, the tip of the puncture portion is provided with an opening in communication with the cavity, and the electrode wire is capable of extending out of the puncture portion through the opening.

20. The interventional device according to claim 19, wherein a through hole in communication with the cavity is provided at a connection between the puncture portion and the stent to allow the electrode wire to pass through.

21. The interventional device according to claim 19, further comprising an electrode wire guiding member accommodated within the cavity, wherein the electrode wire guiding member is configured to guide the electrode wire to extend out of the puncture portion from the opening.

22. The interventional device according to claim 14, wherein the electrode wire extends along an outer side and/or inner side of the stent to the puncture portion.

23. The interventional device according to claim 22, further comprising a fixing layer, wherein a portion of the electrode wire extending along the stent is sandwiched between the fixing layer and the stent.

24. The interventional device according to claim 1, further comprising an occlusion portion, wherein the occlusion portion surrounds at least the puncture portion, and is arranged on an outer surface and/or inner surface of the stent, and the occlusion portion is configured to occlude a gap between the wall and the puncture portion.

25. The interventional device according to claim 24, wherein the occlusion portion comprises a coating layer.

26. The interventional device according to claim 25, wherein during the first state of the stent, at least the tip of the puncture portion is farther away from the stent than the coating layer.

27. The interventional device according to claim 1, further comprising a delivery sheath detachably fitted onto the stent in the first state, wherein the delivery sheath is configured to hold the stent in the first state.

28. The interventional device according to claim 27, wherein an end portion of the delivery sheath near the stent is tearable in a radial direction to be unfolded into a two-dimensional plane.

29. The interventional device according to claim 1, further comprising a balloon, wherein the stent is fitted onto the balloon, and the balloon is configured to enable the stent to switch from the first state to the second state.

30. The interventional device according to claim 1, wherein the tubular body comprises a blood vessel.

31. The interventional device according to claim 1, wherein a material of the stent is selected from any one or a combination of the following: nickel, titanium, nickel-titanium alloy, cobalt, cobalt alloy, chromium, cobalt-chromium alloy, magnesium, magnesium alloy, zinc alloy, and stainless steel.

32. The interventional device according to claim 1, wherein a material of the puncture portion is selected from any one or a combination of the following: nickel, titanium, nickel-titanium alloy, cobalt, cobalt alloy, chromium, cobalt-chromium alloy, magnesium, magnesium alloy, zinc alloy, and stainless steel.

33. The interventional device according to claim 14, wherein a material of the second electrode site is selected from any one or a combination of the following: gold, platinum, iridium, tungsten, magnesium, molybdenum, platinum-iridium alloy, titanium alloy, graphite, carbon nanotubes, poly(3,4-ethylenedioxythiophene) (PEDOT) and its derivative polymers, poly(p-styrene sulfonic acid), polypyrrole, and iridium oxide.

34. The interventional device according to claim 25, wherein a material of the coating is selected from any one or a combination of the following: cellulose, chitin, hyaluronic acid, collagen, gelatin, sodium alginate, polyurethane (PU), poly tetra fluoroethylene (PTFE), expanded PTFE (E PTFE), polylactic acid (PAL), poly(1 lactic acid) (PLLA), poly(D lactic acid) (PDLLA), polyglycolic acid (PGA), polycaprolactone (PCL), polyamide (PA), polyethylene terephthalate (PET), polyether block polyamide (PEBAX), high-density polyethylene (HDPE), thermoplastic polyurethane elastomer (TPU), polyimide (PI), polydimethylsiloxane (PDMS), poly(para-xylylene) (Parylene), epoxy resin, polyamide-imide (PAI), polylactic acid (PHA), poly(lactic-co-glycolic acid) (PHAH), poly(chloro-p-xylylene), SU8, silica gel, and silicone rubber.

35. The interventional device according to claim 10, wherein a material of the metal layer is selected from any one or a combination of the following: gold, platinum, iridium, tungsten, magnesium, molybdenum, platinum-iridium alloy, and titanium alloy.

36. The interventional device according to claim 11, wherein a material of the insulating layer is selected from any one or a combination of the following: polyimide (PI), polydimethylsiloxane (PDMS), poly(para-xylylene) (parylene), polyethylene terephthalate (PET), epoxy resin, polyamide-imide (PAI), polylactic acid (PHA), poly(lactic-co-glycolic acid) (PHAH), poly(chloro-p-xylylene), SU8, silica gel, and silicone rubber.

37. The interventional device according to claim 16, wherein a material of the biodegradable layer is selected from any one or a combination of the following: polylactic acid (PLA), poly(lactic-co-glycolic acid) (PLGA), and magnesium alloy.
